Europäisches Patentamt

(19) European Patent Office

Office européen des brevets

(11) Numéro de publication: **0 092 680**
**B1**

(12) **FASCICULE DE BREVET EUROPÉEN**

(45) Date de publication du fascicule du brevet:
**13.08.86**

(21) Numéro de dépôt: **83102927.7**

(22) Date de dépôt: **07.09.79**

(51) Int. Cl.⁴: **A 23 L 3/34,** A 23 L 1/22

(54) Procédé de préparation de substances antioxygènes et leur utilisation.

(30) Priorité: **29.09.78 CH 10186/78**

(43) Date de publication de la demande:
**02.11.83 Bulletin 83/44**

(45) Mention de la délivrance du brevet:
**13.08.86 Bulletin 86/33**

(84) Etats contractants désignés:
**AT DE FR IT NL**

(56) Documents cité:
**DE-A-2 445 354**
**US-A-3 732 111**
**US-A-3 950 266**

**Le dossier contient des informations techniques présentées postérieurement au dépôt de la demande et ne figurant pas dans le présent fascicule.**

(73) Titulaire: **SOCIETE DES PRODUITS NESTLE S.A.,**
**Case postale 353, CH- 1800 Vevey (CH)**

(72) Inventeur: **Bracco, Umberto, Entre- deux- Crêts 20,**
**CH- 1814 La Tour- de- Peilz (CH)**
Inventeur: **Viret, Jean- Louis, Av. de Jaman 19, CH-**
**1814 La Tour- de- Peilz (CH)**
Inventeur: **Rehacek, Josef, Roselière 13, CH- 1400**
**Yverdon (CH)**

**0 092 680**

## Description

La présente invention concerne un procédé de préparation d'une fraction contenant des substances antioxygènes à partir d'une matière végétale riche en ces substances et l'utilisation de la fraction ainsi obtenue pour préserver les produits alimentaires et cosmétiques de l'oxydation.

Les substances antioxygènes à usage alimentaire général ou cosmétique doivent avoir une bonne activité antioxygène, être stables, incolores, neutres organoleptiquement et leur inocuité doit être garantie.

On connaît des substances antioxygènes obtenues par voie de snythèse dont l'usage est très répandu dans l'industrie alimentaire, généralement des substances phénoliques, par exemple le butylhydroxyanisol (B.H.A.) et le butylhydroxytoluène (B.H.T.) seuls on en mélange synergique. Bien que ces substances aient une activité antioxygène marquée, une bonne stabilité, qu'elles soient incolores et que leur inocuité et leur neutralité vis-à-vis des aliments soient reconnues, leur usage est fortement contesté par nombre de législations alimentaires.

On a également cherché à extraire les substances antioxygènes naturelles contenues dans les matières végétales.

Certains procédés proposent l'extraction directe des substances antioxygènes par voie chimique consistant en un traitement par une solution aqueuse basique.

Ces méthodes utilisant un agent chimique sont également actuellement contestées par les législations alimentaires.

On leur préfère donc les procédés mettant en oeuvre une séparation purement physique des principes antioxygènes. Les extractions classiques de ces substances s'opèrent soit par des solvants organiques, soit par des huiles végétales ou animales. Les méthodes par solvants impliquent la manipulation coûteuse de solvants dangereux souvent difficiles à éliminer et qui peuvent contaminer les substances antioxygènes obtenues. Les solvants ou les huiles présentent en outre l'inconvénient d'extraire également des substances colorantes, comme par exemple la chlorophylle ou des principes aromatiques de la plante qu'il faut éliminer par des traitements supplémentaires de désodorisation et de décoloration Ces traitements diminuent le rendement en principes antioxygènes et augmentent le coût de l'extraction.

Ainsi, un procédé récent décrit dans le brevet des Etats-Unis No. 3,732,111 concerne l'extraction des principes antioxygènes d'épices par broyage fin sur broyer à meules, extraction à l'aide d'une huile végétale à température élevée, séparation par centrifugation et désodorisation par strippage à la vapeur sous vide à température élevée, les principes antioxygènes se retrouvant dans l'huile.

Une autre méthode décrite dans le brevet des Etats-Unis No. 3,950,266 a trait à l'extraction des principes antioxygènes d'épices par traitement d'un poudre d'épice dans un premier stade par solvant à bas point d'ébullition, filtration et séchage, éventuellement décoloration sur charbon actif, filtration et séchage.

Dans un deuxième stade, l'extrait sec est repris par une huile végétale et soumis soit à un strippage sous vide poussé, soit à une distillation moléculaire et ensuite éventuellement purifié par chromatographie en phase liquide.

Nous avons trouvé qu'on peut préparer à partir de matières végétales une fraction contenant des substances antioxygènes naturelles à usage alimentaire et cosmétiques général, et répondant aux exigences mentionnées ci-dessus par un traitement ne faisant pas intervenir d'étapes supplémentaires de décoloration ou désodorisation ou d'élimination de solvants nécessitant la séparation de résidus et donc génératrices de baisse importante du rendement en principes antioxygènes.

Le procédé selon l'invention est caractérisé par le fait

— qu'on prépare une suspension de la matière végétale dans une huile, la matière végétale étant obtenue par extraction par un solvant léger, notamment par l'alcool éthylique non dénaturé suivie d'une évaporation plus ou moins poussée du solvant sous pression réduite,

— qu'on procède à un traitement thermique de la suspension à 200—250°C pendant 1 à 5 minutes ou à 100—200°C pendant 5 à 10 minutes et qu'on sépare le résidu carbonisé avant ou après adjonction d'un vecteur de distillation tel qu'un mono-, di- ou triglycéride à chaîne moyenne et éventuellement standardisation par adjonction d'huile, de sorte que celle-ci contienne 10 à 30 % en poids de matière végétale et 1 à 20 % en poids de vecteur de distillation, et

— qu'on soumet la suspension traitée thermiquement à une distillation moléculaire sous un vide d'au plus 0,67 Pa à une température de 190—280°C et avec un débit de matière de 0,5 à 7 kg/h, et qu'on recueille un condensat contenant les principes antioxygènes, le vecteur de distillation et une partie de l'huile mise en oeuvre.

Par matière végétale, on entend les matières connues pour leur pouvoir antioxygène telles que les épices sous forme de feuilles, fleurs, fruits, racines, rhizômes et en particulier les plantes de la famille des labiacés, romarin, sauge, origan ou marjolaine, thym, etc... et en particulier les résidus de l'extraction des huiles essentielles de sauge et de romarin, par strippage, provenant de l'extraction par un solvant léger, par exemple par l'alcool éhylique non dénaturé des résidus de la distillation des huiles essentielles et séchage plus ou moins prononcé de l'extrait.

Bien qu'on préfère généralement l'éthanol pour réaliser l'extraction, on peut utiliser d'autre solvants

2

ou mélanges de solvants légers c'est-à-dire à bas point d'ébullition. On peut citer le méthanol, le dichloroéthane, l'éther éthylique, l'hexane, le dioxane et les mélanges de ceux-ci entre eux ou avec l'éthanol. Selon l'invention, on réalise l'extraction par un solvant léger, par exemple l'éthanol, de la matière végétale, par exemple des feuilles de romarin strippées et de préférence préalablement broyées. Après séparation du résidu, on procède avantageusement à une évaporation partielle du solvant sous pression réduite jusqu'à ce que l'extrait ait une teneur en matière sèche de 10 à 60 % en poids.

Selon un mode de réalisation préféré, on soumet l'extrait à un mouture très fine ou micronisation. Toute méthode de micronisation peut être utilisée. On préfère utiliser un moulin à billes qui permet un broyage poussé de l'extrait jusqu'à obtenir des particules de 5—20 microns après un ou plusieurs passages. On peut déterminer le degré de désintégration des particules par un choix adéquat de la dimension des billes, de la durée et de l'intensité du traitement.

Selon un mode préféré de mise en oeuvre du procédé, on ajoute 0,5 à 5 % en poids d'eau à la suspension destinée à la micronisation.

L'addition d'une faible quantité d'eau avant la micronisation permet une dispersion extrêmement fine de cette eau dans le mélange obtenu. Cette eau répartie de façon homogène facilite la désodorisation et la décoloration lors de la distillation moléculaire car, étant éliminée avec la fraction de tête de distillation, elle a pour fonction d'entraîner les principes colorants et odorants résiduels.

Pour préparer le mélange destiné à la distillation, la substance végétale, sous forme d'extrait alcoolique, est mélangée avec un vecteur de distillation ou co-distillant tel qu'un mono-, di- ou triglycéride à chaîne moyenne à raison de 1 à 20 % en poids du mélange préparé pour la distillation.

On a constaté que l'adjonction de ces produits avait plusieurs avantages:

— Etant entraînés avec les substances antioxygènes, les vecteurs viennent se déposer sur les condenseurs et permettent l'écoulement de ces substances et la récupération en continu du distillat.
— Lorsque l'on utilise un monoglycéride à pouvoir tensioactif élevé, celui-ci favorise le transfert des principes antioxygènes du solvant léger vers l'huile et ensuite le mélange intime de la fraction distillée contenant les principes antioxygènes, facilitant ainsi la manipulation et le dosage de ceux-ci.
— Ils facilitent la séparation des substances antioxygènes de l'huile ayant servi de solvant car ils sont entraînés avec ces substances.

On ajoute ensuite l'huile au mélange de la substance végétale et du co-distillant en quantité telle qu'elle représente 60 à 80 % en poids du mélange total.

On a constaté qu'on peut avantageusement utiliser directement l'extrait, dont on n'a pas évaporé complètement l'alcool, en mélange avec le co-distillant et l'huile. L'alcool résiduel facilite la décoloration et la désodorisation de la fraction contenant les principes antioxygènes en entraînant les constituants volatils qui sont alors condensés au premier étage de l'appareil de distillation moléculaire.

En variante, on peut réaliser le mélange de l'extrait alcoolique, du co-distillant et de l'huile et procéder à l'évaporation complète de l'alcool sous pression réduite. Selon une autre variante, on peut procéder au prémélange de l'extrait alcoolique et de l'huile, évaporer l'alcool sous pression réduite puis ajouter au mélange obtenu la quantité d'huile et de co-distillant nécessaires.

Tous les mélanges ainsi obtenus subissent avantageusement une homogénéisation à chaud (à 60—70°C lorsque le mélange contient de l'alcool ou à 90—130°C lorsque l'alcool à été préalablement évaporé). On a constaté qu'il était avantageux de procéder à un traitement thermique de la suspension micronisée. On a en effet remarqué la carbonisation d'une fraction représentant environ 5 % en poids de l'extrait sec dans les conditions de température élevée de la distillation.

Pendant la distillation moléculaire, cette fraction a tendance à se déposer sur les condenseurs et souille le condensat. On chauffe la solution ou suspension à environ 200—250°C pendant 1 à 5 min, par exemple dans un échangeur à surface raclée, et on sépare la partie carbonisée, par exemple par tamisage ou centrifugation. La suspension est prête pour la distillation.

En variante, on peut soumettre la suspension à un traitement thermique n'allant pas jusqu'à la carbonisation, par exemple à température de 100—200°C pendant 5—10 minutes, l'opération de tamisage ou centrifugation constituant alors une option.

La distillation moléculaire de la suspension additionnée du vecteur de distillation et éventuellement débarrassée de la fraction susceptible de se carboniser peut avoir lieu dans tout appareil convenable tel qu'un évaporateur à film tombant ou à disque tournant à effet centrifuge. L'échantillon est maintenu en cuve d'attente à 60—90°C pois introduit dans l'appareil.

Si on utilise par exemple un évaporateur à film tombant, celui-ci comporte un premier étage dont la surface d'évaporation est maintenue à 110—180°C, la surface de condensation étant à 80—160°C et dans lequel règne un vide de 0,67 à 26,66 Pa. Le vide peut être produit par une pompe à palettes couplée à des trappes par exemple à azote liquide maintenues à −196°C. Dans ce premier étage s'opère simultanément un dégazage, une décoloration et une désodorisation. Comme indiqué ci-dessus, l'eau qui à été ajouté au mélange sert à entraîner les principes colorants et odorants résiduels et est séparée comme premier condensat.

Dans un deuxième étage dans lequel règne un vide inférieur à 0,67 Pa s'effectue la séparation de la fraction contenant les composés antioxygènes sous forme de condensat, la surface d'évaporation étant

3

# 0 092 680

maintenue à 190—280°C cependant que la surface de condensation est à 80—160°C. Le distillat est constitué essentiellement de l'huile qui a servi comme solvant. Celui-ci peut être recyclé en tête de ligne et servir à nouveau pour la préparation du mélange à distiller, pour la micronisation ou pour la standardisation du mélange micronisé.

Le débit du produit soumis à la distillation moléculaire peut varier de 0,5 à 7 kg/h selon les conditions opératoires.

On a constaté que le rendement de l'extraction des principes antioxygènes est bien supérieur selon l'invention à celui des procédés analogues, car les suspensions soumises à la distillation moléculaire contiennent 5 à 20 % en poids de la matière végétale de départ contre 1 à 2 % en poids, par exemple pour les solutions traitées selon le brevet des Etats-Unis No. 3,950,266.

Selon la variante préférée de traitement d'un extrait alcoolique avec évaporation partielle préalable de l'alcool, la distillation moléculaire s'effectue en une seule étape permettant d'isoler la fraction riche en principes anti-oxygènes tout en effectuant sa décoloration, sa désodorisation et sa séparation de l'huile.

Quelle que soit la matière première mise en oeuvre et son traitement préalable, extraction sans ou avec micronisation, la fraction obtenue contient des principes antioxygènes à pouvoir antioxydant égal ou supérieur aux antioxygènes synthétiques. D'autre part, l'inocuité de celle-ci et sa relative neutralité organoleptique sont remarquables. Le procédé selon l'invention permet une conservation prolongée (d'au moins un an à température ambiante) des principes antioxygènes en solution concentrée (par exemple 40 % en poids) sans aucune diminution du pouvoir antioxydant ni altération organoleptique, ce qui n'est absolument pas le cas des antioxydants chimiques classiques du type B.H.A. ou B.H.T.

L'invention concerne également l'utilisation de la fraction contenant des substances antioxgènes obtenues par le présent procédé pour protéger contre l'oxydation un aliment ou un produit cosmétique. Son incorporation dans les produits peut être réalisée par tout moyen connu, par exemple en solution, suspension ou émulsion dans des solvants, gaz liquéfiés ou autres, c'est-à-dire à l'aide d'un vecteur.

Comme aliments dans lesquels elle peut être incorporée on peut citer les matières grasses telles que les huiles végétales ou les graisses animales, les émulsions du type mayonnaise, pâte à tartiner, bouillons cubes, les produits humides contenant des matières grasses liées tels que la viande, le poisson ou des produits séchés, par exemple lyophilisés, les céréales, farines lactées, poudre de lait entier reconstitué, les légumes secs et particulièrement les flocons de pomme de terre.

Les produits cosmétiques craignant l'oxydation dans lesquels la fraction antioxygène obtenue selon l'invention est avantageusement incorporée sont sous la forme de dispersions aqueuses (lotions, telles que lotions avant ou après rasage), émulsions fluides (laits corporels, laits à démaquiller), crèmes (crème blanche, crème solaire), pâte (masque) etc... Leur protection contre l'oxydation permet en particulier d'éviter les problèmes olfactifs dus au rancissement.

Des résultats satisfaisants sont obtenus par une incorporation de 0,05 à 1 % du condensat soit 0,01 à 0,2 % de substance active entraînable, une partie du condensat étant constituée par l'huile entraînée et le vecteur de distillation.

Selon un mode d'utilisation préféré, on incorpore un mélange broyé du condensat obtenu selon l'invention avec 5 à 40 % calculés sur le poids de condensat d'acide citrique ou ascorbique ou leurs sels et esters. On a en effet constaté qu'on augmentait ainsi de 7 à 12 fois le pouvoir antioxydant tel que mesuré en temps d'induction par la méthode ASTELL.

Les exemples non limitatifs qui suivent illustrent la mise en oeuvre du procédé selon l'invention. Dans ceux-ci, les pourcentages et quantités sont pondéraux sauf indication contraire.

Exemple 1

Des aiguilles de romarin strippées provenant de la distillation d'huile essentielle sont extraites à l'alcool éthylique non dénaturé au reflux et l'extrait séché ou partiellement séché.

15 parties de l'extrait sont mélangées à 85 parties d'huile d'arachide et on ajoute au mélange 1 à 5 % d'eau.

La suspension est traitée dans un moulin à billes DINO à disques agitateurs avec séparateur distant de 0,2 mm et vitesse circonférentielle des disques de 10 m/s, la chambre de broyage étant remplie aux quatre cinquièmes avec des billes de 1 à 1,5 mm de diamètre. La micronisation est effectuée en deux passages avec un temps de résidence de 3,5 min. On obtient ainsi une suspension de viscosité 0,44 P1 mesurée à 25°C.

La suspension micronisée est soumise à un traitement thermique par passage dans un échangeur à surface raclée à 200°C pendant une minute puis passée dans un tamis à mailes de diamètre 0,15 mm pour séparer la partie carbonisée correspondant à environ 5 % de l'extrait sec.

On standardise la suspension huileuse tamisée par adjonction à raison de 3 à 4 parties d'huile d'arachide pour une partie de suspension et on ajoute 5 parties de triglycéride à chaîne moyenne à 95 parties de la suspension standardisée. Celle-ci est placée en cuve d'attente tempérée à 70°C.

La distillation moléculiare de la suspension est effectuée dans un évaporateur LEYBOLD à film tombant de 2 étages, ayant une surface active de l'échangeur de chaleur de 0,1 m² et une vitesse de rotation de 25—600 tours/min dans les conditions suivantes:

4

| débit | 0,5 à 1 kg/h |
|---|---|
| température des trappes | −196°C |
| température de la surface chauffée au premier étage | 120—160°C |
| température du condenseur au 1er étage | 90—150°C |
| vide au 1er étage | 0,67 Pa |
| température de la surface chauffée au 2ème étage | 200—230°C |
| température du condenseur au 2ème étage | 90—150°C |
| vide au 2ème étage | <0,13 Pa |

On recueille le condensat du 2ème étage de couleur ambre clair, de goût et d'odeur très faibles représentant 85 % (calculé sans le co-distillant) de l'extrait alcoolique traité.

Exemple 2
On procède comme dans l'exemple 1 sauf qu'on remplace l'étape de carbonisation par un traitement thermique de la suspension à 130°C pendant 5—10 minutes et on centrifuge à 85°C. On receuille le condensat du 2ème étage de couleur ambre clair, de goût et d'odeur très faibles représentant 60 % de l'extrait alcoolique traité.

Exemples 3—4
Des aiguilles de romarin strippées provenant de la distillation d'huile essentielle sont extraites à l'alcool éthylique non dénaturé, l'extrait est débarrassé des résidus par filtration ou centrifugation et l'alcool évaporé sous pression réduite jusqu'à une teneur en matière sèche de 45 à 50 %. On mélange 10 parties d'extrait à 10 parties de co-distillant (monoglycéride, par exemple DIMODAN S de la maison Grindsted) et à 80 parties d'huile d'arachide. Le mélange est homogénéisé à 60—70°C et traité comme indiqué dans le tableau I ci-dessous;

TABLEAU I

| Exemple | Traitement thermique 2 minutes à 200—250°C | Filtration ou centrifugation | Rendement calculé sans co-distillant en % des feuilles de romarin traitées |
|---|---|---|---|
| 3 | + | − | 15—16 |
| 4 | + | + | 15—16 |

Le mélange est ensuite soumis à la distillation moléculaire comme à l'exemple 1. On recueille le condensat du deuxième étage de goût et d'odeur faibles contenant les principes antioxygènes et représentant le rendement par rapport à l'extrait alcoolique traité indiqué dans le tableau I, tandis que le distillat constitué essentiellement par l'huile d'arachide est recyclé pour être réutilisé.

Exemples 5—6
On mélange 30 parties de l'extrait alcoolique de romarin obtenu comme aux exemples 3—4 à 70 parties d'huile d'arachide et on termine l'évaporation de l'alcool sous pression réduite. 27 parties du mélange obtenu sont ensuite mélangées à 63 parties d'huile d'arachide et 10 parties de co-distillant (monoglycéride, par exemple, DIMODAN S de la maison Grindsted), puis l'ensemble est homogénéisé à 90—130°C pendant 2 minutes. Le mélange est ensuite traité comme indiqué dans le tableau II ci-dessous:

# 0 092 680

### TABLEAU II

| Exemple | Traitement thermique 2 minutes à 200—250°C | Filtration ou centrifugation à 100°C | Rendement calculé sans co-distillant en % des feuilles de romarin traitées |
|---|---|---|---|
| 5 | + | − | 13 |
| 6 | + | + | 13 |

Les opérations ultérieures sont les mêmes que dans les exemples 3—4 et les résultats obtenus analogues.

### Exemples 7—8

On mélange 10 parties de l'extrait alcoolique de romarin obtenue comme aux exemples 3—4 à 80 parties d'huile d'arachide et 10 parties de co-distillant (monoglycéride, par exemple DIMODAN S de la maison Grindsted) et on évapore l'alcool sous pression réduite.

L'ensemble est homogénéisé à 90—130°C pendant 2 minutes puis traité comme indiqué dans le tableau III ci-dessous:

### TABLEAU III

| Exemple | Traitement thermique 2 minutes à 200—250°C | Filtration ou centrifugation 100°C | Rendement calculé sans co-distillant en % des feuilles de romarin traitées |
|---|---|---|---|
| 7 | + | − | 13 |
| 8 | + | + | 13 |

Les opérations ultérieures sont les mêmes que dans les exemples 3—4 et les résultats obtenus analogues.

### Exemple 9

On mesure l'absorption d'oxygène selon la méthode ASTELL de différents condensats obtenus par micronisation ou extraction et distillation moléculaire dans les conditions des exemples 1 et 4. Cette méthode consiste à mesurer l'absorption d'oxygène des graisses et des huiles au cours du temps dans des conditions précises de température et d'enregistrer la période d'induction par rapport à un échantillon de référence. Elle est décrite dans "BFMIRA Tech. Circular No. 487, July 1971, Meora M. L., Rosie D. A., a sensitive oxygen absorption apparatus for study the stability of fats".

Le substrat est 4 g de graisse de poule contenant 1000 ppm d'extrait rapporté à la quantité de substance antioxygène entraînable contenue dans la matière végétale, l'atmosphère au dessus de l'échantillon étant de l'air tandis que la température est de 90°C. Les résultats obtenus sont consignés dans le tableau IV ci-dessous:

### TABLEAU IV

| Nature de l'extrait | Période d'induction (heures) |
|---|---|
| Graisse de poule sans substance antioxygène | 3—4 |
| Graisse de poule +1000 ppm de substance active entraînable du romarin selon l'exemple 1 | 20—25 |
| Graisse de poule +1000 ppm de substance active entraînable du romarin selon l'exemple 4 | 20—25 |
| Graisse de poule +1000 ppm de mélange à poids égal de butylhydroxyanisol (BHA) et de butylhydroxytoluène (BHT) | 20—25 |

### Exemple 10

Une quantité correspondant à 300 ppm de substance active entraînable du romarin obtenue comme décrit dans les exemples 1 à 8 est ajoutée à des flocons de pommes de terre, au cours de la fabrication de la purée de pommes de terre instantanée.

6

L'évolution de la dégradation des lipides contenus dans ce produit est suivie par mesure de la formation de pentane dans l'espace-de-tête, qui provient de la dégradation de l'acide octadécadiénoique présent, la pentane étant déterminé par chromatographie en phase gazeuse selon la méthode décrite par M. Arnaud et J. J. Wuhrmann, "Dehydrated food oxidation as measured by thermal release of hydrocarbons", 4th International Congress of Food Science and Technology, Madrid 23—27.9.1974.

L'efficacité du pouvoir antioxydant des condensats est démontrée dans le tableau V suivant:

TABLEAU V
Flocons de pommes-de-terre

| | sans addition | $UI \times 10^3$ Petane avec addition 300 ppm de substance active entraînable |
|---|---|---|
| Stockage mois à 30°C | | |
| 0 | 0 | 0 |
| 1 | 8 | 0,2 |
| 2 | 17 | 4 |
| 3 | 26 | 8,5 |

Exemple 11

Une quantité de condensat du romarin obtenu comme décrit dans les exemples 1 à 8 correspondant à 500 ppm de substance active entraînable, est ajoutée à une farine de blé pré-cuite avant séchage final. L'évolution de la dégradation lipidique dans le produit est suivie comme à l'exemple précédent et l'efficacité de l'antioxydant est déterminée avec les résultats indiqués dans le tableau VI ci-dessous:

TABLEAU VI
Farine de blé pré-cuite

| | sans addition | $UI.10^5$ Pentane avec addition 500 ppm |
|---|---|---|
| Stockage mois à 30°C | | |
| 0 | 0 | 0 |
| 1 | 3,6 | 1,8 |
| 2 | 8,9 | 2,1 |
| 3 | 13,6 | 4,7 |

Exemple 12

On broye le condensat obtenu selon l'exemple 4 avec 10 % d'acide ascorbique (calculé sur le poids de condensat) sur broyeur à cylindres de granit.

Le pouvoir antioxydant du mélange broyé est mesuré par la méthode ASTELL comme à l'exemple 9.

Pour 0,2 % de mélange, on constate que le temps d'induction est de 140—160 heures, ce qui correspond à une augmentation de 7 à 9 fois du pouvoir antioxydant du condensat seul.

Si on effectue le mélange dans les mêmes proportions sans broyage, le temps d'induction obtenu est de 75 heures. Il y a donc un effet de synergie.

Exemple 13

On prépare une crème blanché, du type émulsion huile dans l'eau par mélange des ingrédients suivants:

| | | | |
|---|---|---|---|
| 1. | Stéaryl éther polyoxyéthylène | | 2 % |
| | Cétyl éther polyoxyéthylène | | 2 % |
| | Monostéarate de glycérol auto émulsionnable | | 4 % |
| | Cosbiol (perhydrosqualène) | | 5 % |
| | Huile de soja | | 5 % |
| | Huile minérale | | 15 % |
| | Acide stéarique | | 2 % |
| | Condensat de l'exemple 4 | | 0,2 % |
| 2. | Triéthanolamine | | 0,4 % |
| 3. | ®Carbopol 941 (Goodrich) | | 4 % |
| | Paraoxybenzoate de méthyle | | 0,3 % |
| | Eau déminéralisée stérile | | Complément à 100 % |
| 4. | Parfum | | Selon désir |

Le mode opératoire consiste à chauffer vers 80—85°C la phase grasse (1), dans laquelle ou aura préalablement ajouté le condensat antioxydant, puis à chauffer à la même température l'eau déminéralisée stérile de la phase (3). Dans celle-ci, on dissout l'agent de conservation puis, la température étant à 70°C, on introduit le Carbopol et on laisse gonfler quelques heures. On fait l'émulsion vers 80—85°C on versant la phase (1) dans la phase (3), dans laquelle on aura préalablement ajouté la phase (2), soit la triéthanolamine. L'émulsion est réalisée par agitation pendant 15 min à l'aide d'une turbine. On refroidit alors à 35°C pour ajouter la phase (4). A 25°C, la crème est terminée.

Cette crème s'avère avoir une action protectrice agréable et une excellente conservation.

Exemple 18

On prépare une crème blanche du type émulsion eau dans l'huile par mélange des ingrédients suivants:

| | | |
|---|---|---|
| Lanolate de magnésium | | 0,9 % |
| Alcool de lanoline | | 8,1 % |
| Huile de paraffine | | 38,7 % |
| Huile d'avocat | | 0,3 % |
| Ozokérite | | 2,0 % |
| Paraoxybenzoate de méthyle | | 0,3 % |
| Condensat selon l'exemple 4 | | 0,2 % |
| Eau déminéralisée stérile | | Complément à 100% |
| Parfum | | Selon désir |

Pour ce faire, on dissout le lanolate de magnésium dans l'huile de paraffine à 100°C environ, puis on refroidit à 80°C avant d'introduire l'alcool de lanoline et l'ozokérite. On refroidit à 40°C pour introduire l'huile d'avocat et le condensat antioxydant, puis on ajoute le milieu aqueux contenant l'agent de

0 092 680

conservation sous forte agitation. Sous agitation lente, on refroidit alors jusqu'à température ambiante et on termine la préparation par adjonction du parfum.

La crème obtenue a une excellente conservation au stockage.

**Revendications**

1. Procédé de préparation d'une fraction contenant des substances antioxygènes à partir d'une matière végétale riche en ces substances, caractérisé par le fait

— qu'on prépare une suspension de la matière végétable dans une huile, la matière végétale étant obtenue par extraction par un solvant léger, notamment par l'alcool éhylique non dénaturé suivie d'une évaporation plus ou moins poussée du solvant sous pression réduite,
— qu'on procéde à un traitement thermique de la suspension à 200—250°C pendant 1 à 5 minutes ou à 100—200°C pendant 5 à 10 minutes et qu'on sépare le résidu carbonisé avant ou après adjonction d'un vecteur de distillation tel qu'un mono-, di- ou triglycéride à chaîne moyenne et éventuellement standardisation par adjonction d'huile, de sorte que celle-ci contienne 10 à 30 % en poids de matière végétale et 1 à 20 % en poids de vecteur de distillation, et
— qu'on soumet la suspension traitée thermiquement à une distillation moléculaire sous un vide d'au plus 0,67 Pa à une température de 190—280°C et avec un débit de matière de 0,5 à 7 kg/h, et qu'on recueille un condensat contenant les principes antioxygènes, le vecteur de distillation et une partie de l'huile mise en oeuvre.

2. Procédé selon la revendication 1, caractérisé par le fait qu'on micronise la suspension avant le traitement thermique de celle-ci.

3. Procédé selon la revendication 1, caractérisé en ce que la matière végétale traitée est constituée de résidus de la distillation de l'huile essentielle de romarin.

4. Procédé selon la revendication 2, caractérisé en ce qu'on ajouté à la suspension 0,5 à 5 % en poids d'eau avant micronisation.

5. Utilisation de la fraction contenant des substances antioxygènes obtenues par le procédé selon l'une des revendications 1 à 4 pour protéger contre l'oxydation un produit alimentaire ou cosmétique, caractérisée par le fait qu'on incorpore de 0,05 à 1 % en poids de cette fraction dans ledit produit.

6. Utilisation selon la revendication 5, caractérisée par le fait qu'on incorpore au produit alimentaire ou cosmétique un mélange broyé de la fraction antioxygène et de 5 à 40 %, calculé sur le poids de la fraction antioxygène, d'acide citrique au ascorbique ou leurs sels et esters.

**Patentansprüche**

1. Verfahren zur Herstellung einer antioxidierend wirkende Substanzen enthaltenden Fraktion aus einem Pflanzenmaterial, das reich an diesen Substanzen ist, dadurch gekennzeichnet,

— daß man eine Suspension des Pflanzenmaterials in einem Öl bereitet, wobei das Planzenmaterial durch Extraktion mittels eines leichten Lösungsmittels, insbesondere durch nichtdenaturierten Ethylalkohol, mit anschließendem weitgehendem oder weniger weitgehendem Eindampfen des Lösungsmittels unter vermindertem Druck erhalten worden ist,
— daß man eine Wärmebehandlung der Suspension bei 200 bis 250°C während 1 bis 5 Minuten oder bei 100 bis 200°C während 5 bis 10 Minuten durchführt, und daß man den verkohlten Rückstand abtrennt, bevor oder nachdem man einen Destillationsträger, wie ein Mono-, Di- oder Triglycerid mittlerer Kettenlänge, dazugegeben hat und sie eventuell durch Hinzufügen von Öl standardisiert hat, sodaß sie 10 bis 30 Gew.-% des pflanzliche Materials und 1 bis 20 Gew.-% des Destillationsträgers enthält, und
— daß man die wärmebehandelte Suspension einer Molekulardestillation unter einem Vakuum von höchstens 0,67 Pa bei einer Temperatur von 190 bis 280°C und mit einem Massendurchsatz von 0,5 bis 7 kg je Stunde unterwirft, und daß man ein Kondensat auffängt, das die oxidationsverhindernden Prinzipien, den Destillationsträger und einen Teil des verwendeten Öles enthält.

2. Verfahren nach Anspruch 1, dadurch gekennzeichnet, daß man die Suspension vor ihrer Wärmebehandlung mikronisiert.

3. Verfahren nach Anspruch 1, dadurch gekennzeichnet, daß das behandelte pflanzliche Material aus Rückständen der Destillation von ätherischem Öl aus Rosmarin besteht.

4. Verfahren nach Anspruch 2, dadurch gekennzeichnet, daß man der Suspension 0,5 bis 5 Gew.-% Wasser vor der Mikronisation zusetzt.

5. Verwendung der die nach einem der Ansprüche 1 bis 3 erhaltenen, oxidationsverhindernde Substanzen enthaltenden Fraktion zum Schützen eines Lebensmittelproduktes oder kosmetischen Produktes vor Oxidation, dadurch gekennzeichnet, daß man 0,05 bis 1 Gew.-% dieser Fraktion in das genannte Produkt einarbeitet.

6. Verwendung nach Anspruch 5, dadurch gekennzeichnet, daß man in das Lebensmittelprodukt oder

9

0 092 680

kosmetische Produkt eine gemahlene Mischung aus der oxidationsverhindernden Fraktion und 5 bis 40 % Zitronen- oder Ascorbinsäure oder ihrer Salze und Ester, bezogen auf das Gewicht der oxidationsverhindernden Fraktion, einarbeitet.

**Claims**

1. A process for the production of a fraction containing oxidation-inhibiting substances from a vegetable material rich in these substances, characterised in that

— the vegetable material is extracted with a light solvent, particularly undernaturated ethyl alkohol and the solvent is partially or almost completely evaporated under reduced pressure and the extract is suspended in an oil,
— the suspension is subjected to a heat treatment at 200—250°C during 1 to 5 min. or 100—200°C during 5 to 10 min. and the carbonized residue is separated, before or after having added a distillation vehicle such as mono-, di- or triglyceride of medium chain length and optionally having added oil thereto so that it contains 10 to 30% by weight of vegetable material and 1 to 20% by weight of distillation vehicle, and
— the heat treated suspension is subjected to molecular distillation under a vacuum of at most 0.67 Pa at a temperature of 190—280°C and with a throughput of material of 0.5 to 7 kg/h, and a condensate containing the oxidation-inhibiting principles, the distillation vehicle and some of the oil used is collected.

2. A process as claimed in claim 1, characterised in that the suspension is micronised before being subjected to the heat treatment.
3. A process as claimed in claim 1, characterised in that the vegetable material treated is formed by residues from the distillation of the essential oil of rosemary.
4. A process as claimed in claim 2, characterised in that from 0.5 to 5% by weight of water is added to the suspension before micronisation.
5. The use of the fraction containing oxidation-inhibiting substances obtained by the process as claimed in anyone of claims 1 to 4 for preserving a food or cosmetic product from oxidation characterised in that 0.05 to 1% by weight of said fraction is incorporated therein.
6. The use as claimed in claim 5, characterised in that a ground mixture of the oxidation-inhibiting fraction with 5 to 40%, based on the weight of the oxidation-inhibiting fraction, of ascorbic or citric acid or their salts and esters is incorporated therein.